# EUROPEAN PATENT SPECIFICATION

(11) **EP 2 568 899 B1**
(45) Date of publication and mention of the grant of the patent: **04.01.2017**
(21) Application number: 11726248.5
(22) Date of filing: 09.05.2011
(51) Int. Cl.: A61B 17/86, A61B 17/88, A61B 17/72, A61B 17/90

(54) **BONE SCREW ASSEMBLY AND INSTRUMENTS FOR IMPLANTATION OF THE SAME**
KNOCHENSCHRAUBENANORDNUNG UND INSTRUMENTE ZU IHRER IMPLANTATION
ENSEMBLE VIS D'OS ET INSTRUMENTS POUR SON IMPLANTATION

(30) Priority: 13.05.2010 US 334234 P
(43) Date of publication of application: 20.03.2013
(73) Proprietor: Synthes GmbH, 4436 Oberdorf (CH)
(72) Inventor: OVERES, Tom, CH-4513 Langendorf (CH); ZURSCHMIEDE, Silas, CH-4513 Langendorf (CH); FLURI, Daniel, CH-4513 Langendorf (CH)
(74) Representative: Orr, Robert
(86) International application number: PCT/US2011/035760
(87) International publication number: WO 2011/143116

(56) References cited:
- WO-A1-2010/065855
- WO-A2-2009/120852
- WO-A2-2011/100512
- DE-U1- 20 314 297
- JP-A- 2001 252 283

## Description

The present invention generally relates to a system comprising a bone screw and an aiming guide for drilling a hole in a bone. More particularly, the invention relates to a system with a bone screw including a second screw.

In the field of orthopedic surgery bone fixation devices using bone screws are commonly used. These bone fixation devices include bone plates, intervertebral implants or intramedullary nails by means of which two or more bones or bone fragments are fixed relative to each other. Typically, the bone fixation devices comprise bone anchors mostly in the form of bone screws, pins or nails by means of which the bones or bone fragments are fixed to the bone plate, intervertebral implant or intramedullary nail and consequently fixed relative to each other. One problem that can arise in case of the above mentioned bone fixation devices is that the bone screws, pins or nails can for instance become dislodged in the bone or in the bone plate, intervertebral implant or intramedullary nail during normal movements of the patient.

From WO 2009/120852 a universal anchor for attaching objects to bone tissue is known. The document discloses an anchoring device for attaching an object to a bone. The device comprises an anchoring member having proximal and distal ends, the proximal end adapted to hold the object to the bone while the distal end is in the bone. The device also comprises a locking member having proximal and distal ends, the proximal end adapted to secure the anchoring member into the bone and to oppose its pull-out or loosening, by stopping its backing or preventing its unscrewing while the distal end is in the bone. First and second fasteners are disclosed, the first fastener adapted to fit to the proximal end of the anchoring member, and the second fastener adapted to fit to the proximal end of the locking member. The end of the second fastener can have an angle that matches an angle of the locking member.

Document WO 2010/065855, which was published after the priority date of the present invention, is directed to an anchor-in-anchor system for use in bone fixation. The fixation system includes a first bone anchor having a shaft for fixation to underlying bone, and a head that defines an internal bore. A second bone anchor extends through the bore and into underlying bone. The head of the first anchor includes an annular body that defines a radially inner surface, an opposing radially outer surface, a proximal end and a distal end. The annular body includes a plurality of circumferentially spaced retention tabs that extend up from the distal end. Thus, terminal ends of the retention tabs are disposed at the proximal end of the head. The retention tabs are configured such that circumferentially adjacent tabs are separated by a slot that extends distally into the proximal end of the head in a direction toward, but not through, a base of the head. The head further defines a bore extending centrally through the annular body along a central bore axis. The central axis extends in a direction angularly offset with respect to the longitudinal axis. The shaft is coupled to the base, and extends radially outward and down from the radially outer surface of the base such that the shaft does not interfere with the bore. The head includes a plurality of helical threads in the bore that extend radially inward from the radially inner surface of the annular body, including the tabs and the base. The central axis of the bore intersects with the longitudinal axis of the shaft so as to define an acute angle. The bore of the first bone anchor is configured to receive the second bone anchor, such that the first and second bone anchors can be fastened together.

Thus, there remains a need for an improved bone anchor device for use in bone fixation that allows to drill a hole for a securing screw into a bone under a particular angle with respect to the axis of a bone fastener and to insert the securing screw in a guided manner.

### Summary of the Invention

The present invention relates to a system as defined in claim 1, which comprises a bone screw and an aiming guide. Preferred embodiments are defined in the dependent claims.

### Brief Description of the Drawings

An exemplary embodiment of the present invention will be described in the following by way of example and with reference to the accompanying drawings in which:
Fig. 1 illustrates a longitudinal section of an embodiment of the bone screw according to the invention;
Fig. 2 illustrates a lateral view of the embodiment of the bone screw of Fig. 1;
Fig. 3 illustrates a perspective view of an embodiment of the screwdriver according to the invention;
Fig. 4 illustrates a longitudinal section of the embodiment of the screwdriver of Fig. 3;
Fig. 5 illustrates a lateral view of the embodiment of the screwdriver of Fig. 3;
Fig. 6 illustrates a lateral view of the embodiment of the screwdriver of Fig. 3 which is orthogonal to the lateral view of Fig. 5;
Fig. 7 illustrates a lateral view of another embodiment of the screwdriver according to the invention;
Fig. 8 illustrates a lateral view of the embodiment of the screwdriver of Fig. 7 which is orthogonal to the lateral view of Fig. 7;
Fig. 9 illustrates a partial section through the embodiment of the screwdriver of Figs. 7 and 8 and a bone screw attached thereto;
Fig. 10 illustrates a perspective view of an embodiment of the aiming guide according to the invention;
Fig. 11 illustrates a longitudinal section through the embodiment of the aiming guide of Fig. 10 and a bone screw coaxially attached thereto;
Fig. 12 illustrates a longitudinal section through the embodiment of the aiming guide of Fig. 10 and a bone screw attached thereto under the angle α;
Fig. 13 illustrates a longitudinal section through the embodiment of the aiming guide of Fig. 10 and a bone screw attached thereto under the angle α and together with a drill guide inserted in the aiming guide and a drill bit;
Fig. 14 illustrates a section through the aiming device and the drill guide of Fig. 13;
Fig. 15 illustrates a longitudinal section through the embodiment of the aiming guide of Fig. 10 and a bone screw attached thereto under the angle α and together with a second screw inserted in the aiming guide;
Fig. 16 illustrates a longitudinal section through the embodiment of the aiming guide of Fig. 10 and a bone screw attached thereto under the angle α and together with a second screw firmly secured in the through hole in the screw head of the bone screw; and
Fig. 17 illustrates an intramedullary nail together with a bone screw and a second screw according to an embodiment of the method for bone fixation according the invention.
Fig. 18 illustrates a lateral view of a system according to an alternate embodiment of the present invention, in a first configuration.
Fig. 19 illustrates a lateral view of the system of Fig. 18, in a second configuration.
Fig. 20 illustrates a cross-sectional lateral view of the system of Fig. 18, in the second configuration.
Fig. 21 illustrates an enlarged cross-sectional lateral view of a portion of the system of Fig. 18.

### Detailed Description

The present invention may be further understood with reference to the following description and the appended drawings, wherein like elements are referred to with the same reference numerals. The present disclosure relates to bone screw assemblies and instruments for implantation of the same as well as to an associated method for implantation of the bone screw assembly using the instruments. In particular, the disclosure relates to a system and method facilitating implantation of a first bone screw, including a through hole extending through a head portion thereof along a through hole axis, and a second screw inserted into the through hole along the through hole axis such that the first and second bone screws are implanted into a bone in a stable configuration.

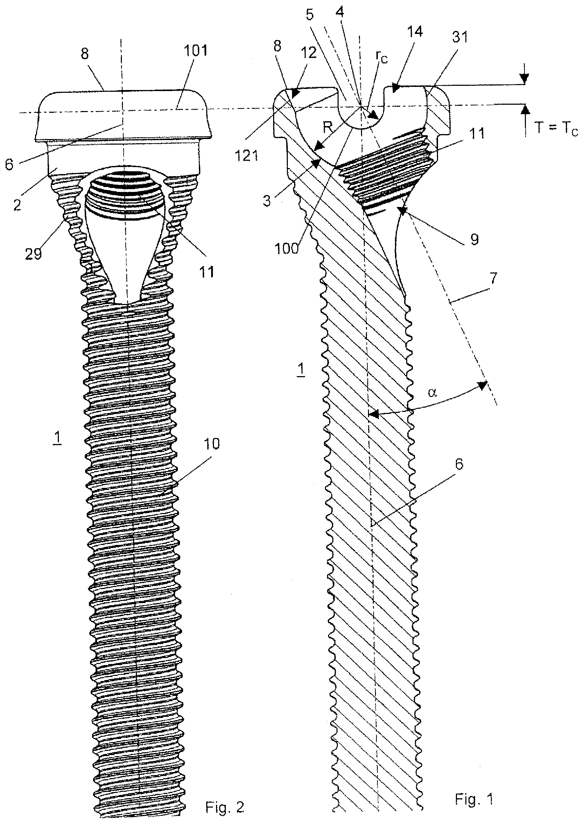

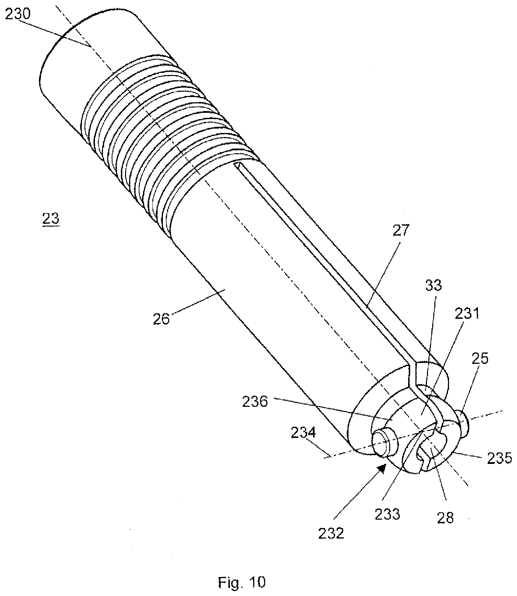

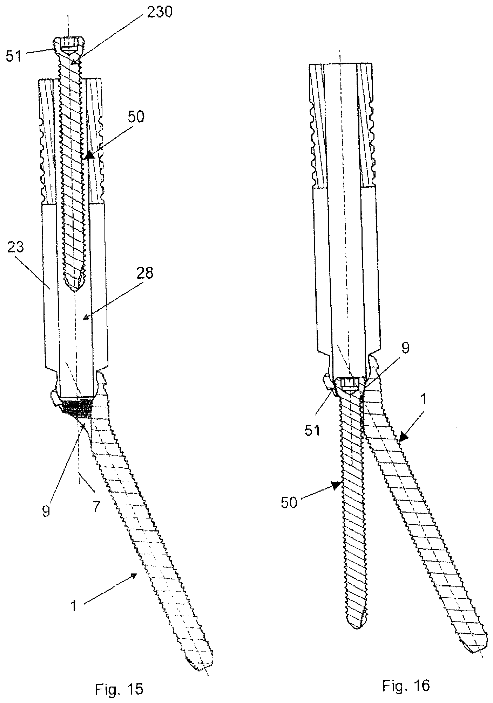

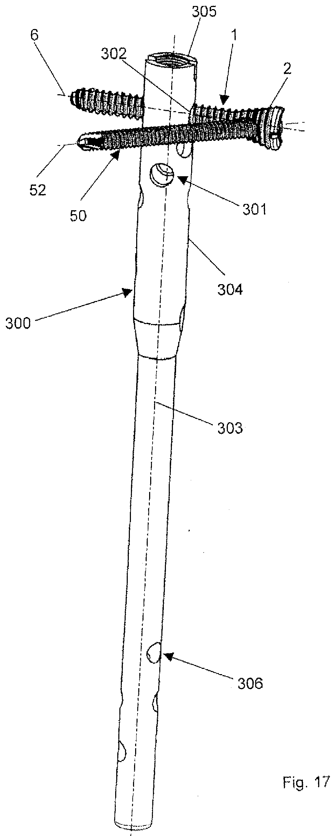

Figs. 1 and 2 illustrate an embodiment of the bone screw 1 with a screw head 2 comprising a conical external thread 29. The bone screw 1 includes a screw axis 6, a threaded shaft 10, a screw head 2 and a rear end 8 at a proximal end thereof. The screw head 2 comprises a through hole 9 penetrating through the screw head 2 and having a through hole axis 7 cutting the screw axis 6 under an acute angle α. The through hole axis 7 cuts the screw axis 2 at a depth T measured from the rear end 8 of the bone screw 1. The through hole 9 has a conical internal thread 11. A second screw 50 (Figs. 16 and 17) can be inserted into the through hole 9 coaxially to the through hole axis 7. The second screw 50 has a conically threaded head 51 which is engagable with the conical internal thread 11 in the through hole 9. The screw head 2 includes a concave seat 14 for releasably coupling a surgical instrument or tool to the bone screw 1. The concave seat 14 comprises a transverse channel 5 and a centrally located recess 3. The transverse channel 5 comprises a channel axis 101 located at a depth T_{C} measured from the rear end 8 of the bone screw 1 and diametrically extending across the screw head 2. The channel axis 101 cuts the screw axis 6 through the point where the through hole axis 7 cuts the screw axis 6. Further, the transverse channel 5 is open at the rear end 8 of the bone screw 1 and the transverse channel 5 has a U-shaped cross-section with a semicircular bottom 100 orthogonal to the channel axis 101. The semicircular bottom 100 has a radius of curvature r_{C} wherein a center of an edge the semicircular bottom 100 of the transverse channel 5 is located on the channel axis 101. The depth T_{C} is equal to the depth T of the point where the through hole axis 7 cuts the screw axis 1. The semicircular bottom 100 defines a seat coaxially to the recess 3 for rotatably receiving cylindrical pins 25 of an aiming guide 23 (Fig. 10) which have a pin diameter equal to twice the radius of curvature r_{C} of the semicircular bottom 100 of the transverse channel 5.

The recess 3 has a spherical shape with a radius of the sphere R and a centre 4 coinciding with the point at which the screw axis 6 and the through hole axis 7 intersect. So the recess 3 forms a pivot bearing for rotatably supporting and guiding a complementarily spherically shaped male connector 232 of an aiming guide 23 (Fig. 10). Due to the facts that the channel axis 101 cuts the screw axis 6 at the point where the centre 4 of the spherically shaped recess 3 is located on the screw axis 6 and that the pivot pins 25 of the aiming guide 23 fit in the transverse channel 5 rotatably about the channel axis 101 the polyaxial pivot bearing formed by the ball-and-socket joint is limited to an uniaxial pivot bearing. When the aiming guide 23 is coupled to the bone screw 1 the aiming guide 23 can only pivot about the channel axis 101 which is orthogonal to a plane defined by the screw axis 6 and the through hole axis 7 of the through hole 9 for the second screw 50. This allows to position the aiming guide 23 in a first position coaxial to the screw axis 6 of the bone screw 1 and in a second position coaxial to the through hole axis 7 of the through hole 9 for the second screw 50. Thus, it will be understood by those of skill in the art, that the second screw 50 may be precisely inserted into the through hole 9 along through hole axis 7, increasing a stability of the screws 1, 50 in situ.

Furthermore, the recess 3 has a constriction 31 at the rear end 8 of the bone screw 1 so that the recess 3 forms a female connector for a snap-lock connection. Additionally, the recess 3 includes a depression 12 which forms a wall portion 121 with the shape of a surface section of a circular cylinder with a radius r ≤ R. The axis of the circular cylinder coincides with the through hole axis 7 of the through hole 9. The depression 12 forms a stop for the rotation of an aiming guide 23 about the channel axis 101 when the aiming guide 23 is coupled to the screw head 2 of the bone screw 1. By means of the stop the aiming guide 23 can be exactly aligned with the through hole 9.

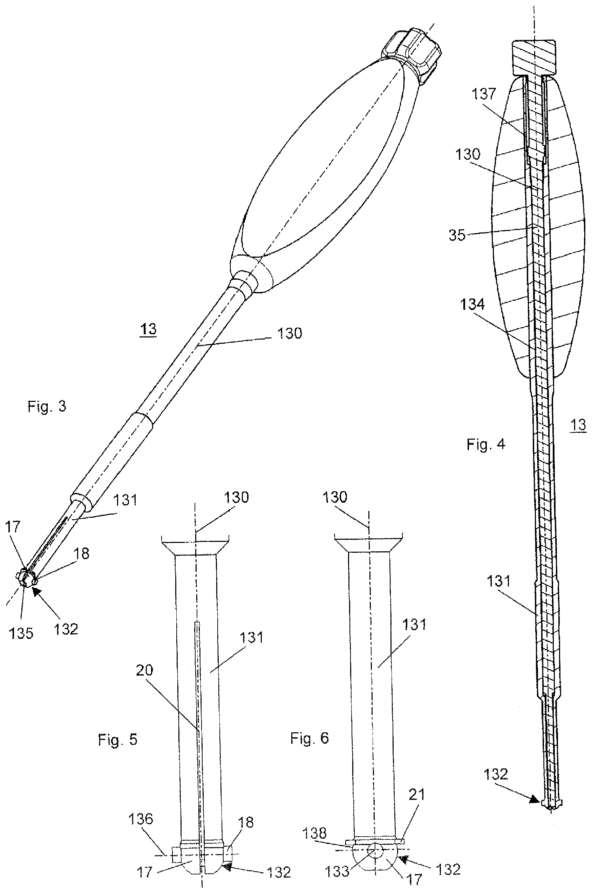

Figs. 3 to 6 illustrate an embodiment of the screwdriver 13 to be used with the bone screw 1 according to Figs. 1 and 2. The screwdriver 13 comprises a longitudinal axis 130, a shaft 131, a front end 135 at a distal end thereof and a male connector 132 which can be coupled to the above embodiment of the bone screw 1. In order to releasably couple the screwdriver 13 to the bone screw 1 the connector 132 is essentially complementarily formed to the concave seat 14 in the screw head 2 of the bone screw 1. The connector 132 includes a partially spherical tip 17 the cross-sectional area of which in a plane perpendicular to the longitudinal axis decreases toward the shaft 131 (i.e., toward the front end 135). Thus, a cross-sectional area of the spherical tip 17 at an end adjacent to the shaft 131 and at the front end 135 is smaller than a cross-sectional area of a mid-section of the spherical tip 17. The tapering of the spherical tip 17 towards the shaft 131 forms a curved contact shoulder 138 which abuts the constriction 31 of the recess 3 at the rear end 8 of the bone screw 1. The screwdriver 13 further comprises a longitudinal slot 20 open at the front end 135 to form the tip 17 as an elastic male connector for a snap-lock connection between the tip 17 and the recess 3 in the screw head 2 of the bone screw 1. The longitudinal slot 20 is arranged orthogonal to a plane defined by the longitudinal axis 130 and the central axis 136 and penetrates through the shaft 131. The connector 132 further includes two driving protrusions 18 extending laterally from the spherical tip 17 in either direction and which are coaxially arranged on a central axis 136. The driving protrusions 18 are circular-cylindrically shaped with a cylinder axis coinciding with the central axis 136. The connector 132 additionally comprises an axial stop 21 which is located between the shaft 131 and the connector 132 at a distance T measured from the central axis 136 towards the shaft 131. The axial stop 21 abuts the rear end 8 of the bone screw 1 allowing to keep the screwdriver 13 exactly coaxially to the screw axis 6 of the bone screw 1. The shaft 131 and the connector 132 comprise a coaxial through bore 134 with an internal thread 137 for engaging an external thread arranged on a locking pin 35 (Fig. 4) which is insertable in the through bore 134 in such a manner that the locking pin 35 can be advanced towards the front end 135 of the screwdriver 13 in order to prevent the tip 17 from radially collapsing so that it can be firmly kept in the recess 3 in the screw head 2 of the bone screw 1.

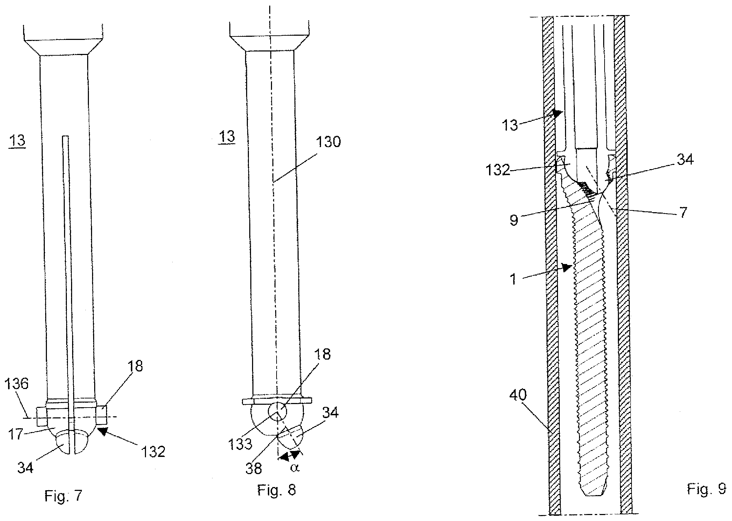

The embodiment of the screwdriver 13 illustrated in Figs. 7 to 9 differs from the embodiment of Figs. 3 to 6 only therein that the male connector 132 further includes a nose 34 extending from the tip 17 in a direction towards the front end 135. The nose 34 has a nose axis 38 extending under the angle α with respect to the longitudinal axis 130 of the screwdriver 13. Thus, the screwdriver 13 can be coupled to the bone screw 1 in only one rotative position, namely the one position where the nose 34 engages the through hole 9 in the bone screw 1 in such a manner that the nose axis 38 coincides with the through hole axis 7.

Fig. 10 illustrates an embodiment of the aiming guide 23 to be used with the bone screw 1 according to Figs. 1 and 2. The aiming guide 23 comprises a longitudinal axis 230, a coaxial through bore 28, a guide sleeve 26, a front end 235 and a male connector 232 terminally arranged at the front end 235. To releasably couple the aiming guide 23 to the bone screw 1 the connector 232 is essentially complementarily formed to the seat 14 in the screw head 2 of the bone screw 1. The connector 232 includes a spherically shaped tip 231 which tapers inward toward the front end 235 and toward the guide sleeve 26 forming a ball-and-socket joint with the recess 3 of the above described embodiment of the bone screw 1. The spherically shaped tip 231 has a radius of the sphere R and a centre 233 located on the longitudinal axis 230. Additionally, the connector 232 includes two pins 25 diametrically projecting over the tip 231 in either direction and which are coaxially arranged on a central axis 234 which extends orthogonal to the longitudinal axis 230 and which cuts the longitudinal axis 230 through the centre 233 of the spherically shaped tip 231. The pins 25 are circular-cylindrically shaped with a cylinder axis coinciding with the central axis 234 so as to form axles coaxially and rotatably insertable in the transverse channel 5 in the screw head 2 of the bone screw 1. The aiming guide 23 further comprises a longitudinal slot 27 open at the front end 235 to form the tip 231 as an elastic male connector for a snap-lock connection between the aiming guide 23 and a the spherical recess 3 in the screw head 2 of the bone screw 1. The longitudinal slot 27 is arranged orthogonal to a plane defined by the longitudinal axis 230 and the central axis 234 and penetrates through the guide sleeve 26. Between the tip 231 and the guide sleeve 26 a cylindrical collar 33 is arranged coaxially to the longitudinal axis 230 and which has a radius r ≤ R.

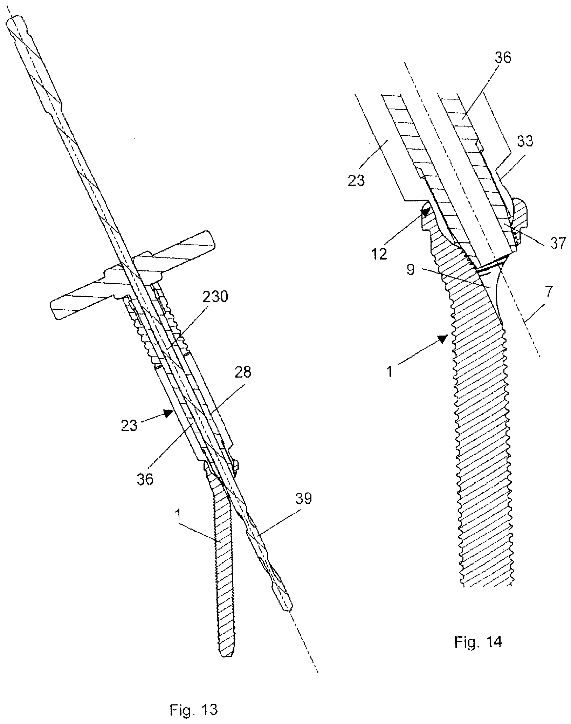

As illustrated in Figs. 13 and 14 a drill guide 36 can be inserted in the through bore 28 in the aiming guide 23. The drill guide 36 has a conical tip 37 which fits into the tapered through hole 9 in such a manner that the drill guide 36 is exactly aligned with the through hole axis 7 of the through hole 9 in the bone screw 1.

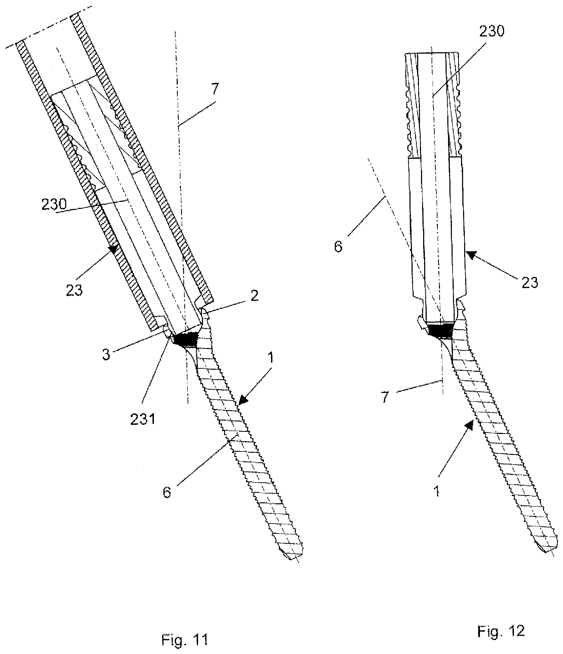

Figs. 11 to 17 show a method for bone fixation by using an intramedullary nail 300 and bone screws 1 along with second screws 50, which are briefly described in the following section. The intramedullary nail 300 comprises a nail axis 303, a proximal end 305, a peripheral surface 304, a number of proximal locking holes 301 with a most proximal locking hole 302 and a number of distal locking holes 306. The proximal and distal locking holes 301, 306 extend transverse to the nail axis 303. The intramedullary nail 300 is inserted into the intramedullary canal of a long bone in such a manner that the portion of the intramedullary nail 300 containing the distal locking holes 306 is located in a distal bone fragment and the portion containing the proximal locking holes 301 is located in the proximal bone fragment. In order to lock the intramedullary nail 300 in the bone a bone screw 1 each is driven through all or a number of selected proximal and distal locking holes 301, 306. Using the bone screw 1 according to the invention the bone screws 1 can be driven through all or the selected proximal and distal locking holes 301, 306 and the second screws 50 can be anchored in the bone. It should be appreciated that instead of driving the bone screws 1 into the proximal and distal locking holes 301, 306 the second screws 50 could be driven into the proximal and distal locking holes 301, 306 and the bone screws 1 could be anchored in the bone.

The method for inserting the bone screws 1 into the nail 300 and anchoring the second screws 50 into the bone comprises the steps of making an incision into the tissue surrounding a bone to be treated and positioning an intramedullary nail 300 in the bone. An aiming device (not shown) to the proximal end 305 of the intramedullary nail 300, wherein the aiming device has guide bores for inserting guide sleeves and/or tissue protection tubes 40 coaxially to each of all or of a number of selected proximal and/or distal locking holes 301, 306. A tissue protection tube 40 (Figs. 9 and 11) is inserted into a selected guide bore in the aiming device coaxially to one of the proximal and distal locking holes 301, 306 until the front end of the tissue protection tube 40 contacts the surface of the bone. A first bore hole is then drilled into a bone for insertion of a bone screw 1 through the selected proximal or distal locking hole 301, 306 by using the aiming device, wherein the first bore hole is aligned with the selected proximal or distal locking hole 301, 306. Further, the bore hole extends on either side of the intramedullary nail 300 in such a manner that a bone screw 1 can penetrate through the selected proximal or distal locking hole 301, 306 of the intramedullary nail 300 when the bone screw 1 is anchored in the bone. The bone screw 1 is coupled to the connector 132 of the screwdriver 13 by using the snap-lock connection between the bone screw 1 and the screwdriver 13 and advancing the bone screw 1 through the tissue protection tube 40. The bone screw 1 is then screwed into the bone using the screwdriver 13. Once the bone screw 1 is screwed into the bone, the screwdriver 13 may be removed. The above-described steps may be repeated until a bone screw 1 has been inserted into all of the desired proximal and/or distal locking holes 301, 302.

The aiming guide 23 is then inserted through the protection tube 40, as shown in Fig. 11, to attach the aiming guide 23 to the bone screw 1 using the snap-lock connection between the tip 231 of the aiming device 23 and the recess 3 in the screw head 2 of the bone screw 1 such that the longitudinal axis 230 of the aiming guide 23 is aligned with the screw axis 6 of the bone screw 1. The tissue protection tube 40 and the aiming device may be removed and the and the aiming guide 23 pivoted about the central axis 234 defined by the pins 25 arranged at the connector 232 of the aiming guide 23 until the collar 33 of the aiming guide 23 abuts a stop in the recess 3 in the screw head 2 of the bone screw 1 so that the longitudinal axis 230 of the aiming guide 23 is aligned with the through hole axis 7 of the through hole 9 in the bone screw 1 (Fig. 12). The stop is formed by the wall portion 121 of the depression 12 in the recess 3 in the screw head 2 of the bone screw 1. The drill guide 36 is then inserted into the through bore 28 in the aiming guide 23 (Fig. 13) and a second bore hole is drilled into the bone using the drill guide 36 as a guide for the drill bit 39. Once the second bore hole has been drilled, the drill guide 36 is removed and the second screw 50 is inserted through the through bore 28 in the aiming guide 23 (Fig. 15) and advanced through the second screw 50 into the bone (Fig. 16). The aiming guide 23 may then be removed and the steps described above repeated until a second screw 50 each is anchored in the bone passing through the through hole 9 of each of the bone screws 1. Once all of the desired bone screws 1 and second screws 50 have been inserted into the bone, the incision may be closed.

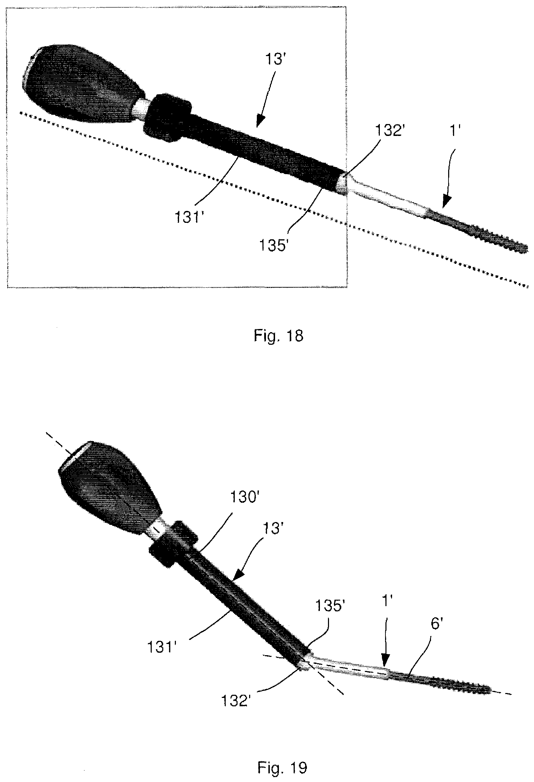

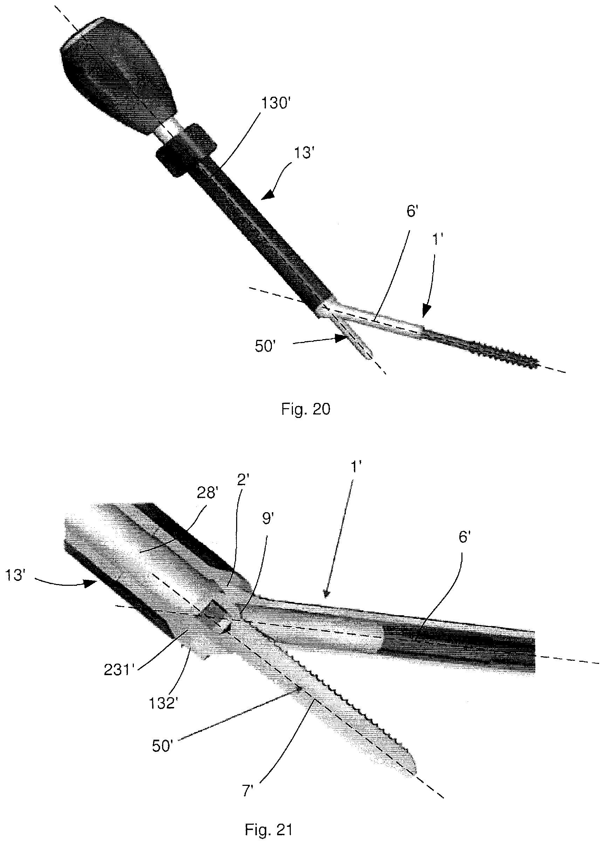

As shown in Figs. 18 - 21, an alternate embodiment of the assembly of the present invention is substantially similar to the assembly described above in regard to Figs. 1 - 17, comprising a first bone screw 1', a second bone screw 50' and a screwdriver 13'. The screwdriver 13', however, combines elements of the screwdriver 13 and the aiming guide 23, as described above, such that two separate devices are not required for insertion of the first bone screw 1' and for aiming the second bone screw 50'. In addition, the screwdriver 13' includes a connector 132' at a distal end 135' thereof, which extends around a head 2' of the first bone screw 1' rather than within a recess thereof.

The first bone screw 1' extends along a first axis 6' and includes a head 2', which has an exterior surface that is at least partially spherical. The exterior surface may also include portions that are substantially planar permitting a torsional force to be applied thereto via the screwdriver 13'. Similarly to the bone screw 1, the first bone screw 1' includes a through hole 9' extending along a second axis 7' to receive the second screw 50' therein. The second screw 50' is substantially similar to the second screw 50' described above.

The screwdriver 13' includes a shaft 131' extending along a longitudinal axis 130' with a connector 132' formed at the distal end 135' thereof. The screwdriver 13' also includes a channel 28' extending therethrough along the longitudinal axis 130' sized and shaped to permit the second bone screw 50' to be inserted therethrough. The connector 132' includes a partially spherical interior surface 231' sized and shaped to receive the head 2' of the first bone screw 1' therein. In one embodiment, the connector 132' may be keyed (e.g., include planar portions corresponding to the planar portions of the head 2') permitting the screwdriver 13' to apply torsional forces to the bone screw 1' while also permitting the first bone screw 1' to pivot with respect to the screwdriver 13' via the partially spherical surfaces of the connector 132' and the head 2'. The interior surface 231' may receive the head 2' via, for example, a snap fit.

In an alternative embodiment, the head 2' may include pins extending radially outward therefrom, which are substantially similar to the pins 25 of the connector 232 of the aiming guide 23, and the connector 132' may include a transverse channel diametrically extending thereacross similarly to the channel 5 of the bone screw 1, as described above. It will be understood by those of skill in the art that such a configuration also permits the first bone screw 1' to be rotated via the screwdriver 13' while also permitting the first bone screw 1' to be pivoted relative thereto.

The first and second bone screws 1', 50' and the screwdriver 13' may be used in a manner substantially similar to the method described above. In particular, the first bone screw 1' may be inserted into a desired one of the proximal and/or distal locking holes 301, 306 of an intramedullary nail 300 inserted into the bone. A first bore hole may be drilled through the desired one of the first and second holes 301, 306 to accommodate the first bone screw 1'. As described above, the screwdriver 13' is coupled to the first bone screw 1' by receiving the head 2' within the connector 132'. In an initial configuration, the longitudinal axis 130' of the screwdriver 13' is coaxially aligned with the first axis 6' of the bone screw 1'. The first bone screw 1' is screwed into the desired one of the holes 301, 306 and the first bore hole via the screwdriver 13'. Once the first bone screw 1' has been inserted, as desired, the screwdriver 13' is pivoted with respect to the first bone screw 1' about the head 2' until the channel 28' thereof is coaxially aligned with the second axis 7' of the through bore 9'. A second bore hole may be drilled into the bone through the channel 28' and through bore 9' to accommodate the second bone screw 50'. The second bone screw 50' may then be guided through the channel 28' and into through bore 9' to be advanced into the second bore hole in the bone. It will be understood by those of skill in the art that the above-described steps may be repeated, as desired, until a desired number of first and second bone screws 1', 50' have been inserted into the bone.

The scope of the present application is not intended to be limited to the particular embodiments of the process, machine, manufacture, composition of matter, means, methods and steps described in the specification. As one of ordinary skill in the art will readily appreciate from the disclosure of the present invention, processes, machines, manufacture, composition of matter, means, methods, or steps, presently existing or later to be developed that perform substantially the same function or achieve substantially the same result as the corresponding embodiments described herein may be utilized according to the present invention.

It will be appreciated by those skilled in the art that various modifications and alterations of the invention can be made without departing from the broad scope of the appended claims. Some of these have been discussed above and others will be apparent to those skilled in the art.

## Claims

1. A system comprising a bone screw (1) and an aiming guide (23) for drilling a hole in a bone;
in which the bone screw (1) comprises:
a screw shaft (10) extending longitudinally along a screw axis (6);
a screw head (2) extending from a proximal end of the screw shaft (10); and
a through hole (9) defining a through hole axis (7) and extending through the screw head (2), the through hole axis (7) intersecting the screw axis (6) at an acute angle α, the through hole (9) being adapted to receive therein a second screw (50) and tapering from a first end at a proximal end of the screw head (2) to a second end opening to an outer surface of the bone screw (1);
in which the screw head (2) has a concave seat (14) comprising a channel (5) and a centrally located recess (3), the channel (5) disposed at the first end of the through hole (9) and extending from the outer surface of the bone screw (1) transversely through the screw head (2) along a channel axis (101) that is substantially perpendicular to the screw axis (6), the channel (5) being open at the proximal end of the bone screw (1);
in which the aiming guide (23) is for drilling a hole having an axis which coincides with the through hole axis (7) of the through hole (9) in the screw head (2) of the bone screw (1);
and in which the aiming guide (23) comprises:
a guide sleeve (26);
a coaxial through bore (28); and
a male connector (232) terminally arranged at a front end (235) of the aiming guide (23) for coupling to the recess (3) in the screw head (2), the connector (232) including a tip (231) a width of which decreases towards the front end (235) of the aiming guide (23) and two pins (25) diametrically projecting over the tip (231) in either direction and coaxially arranged on a central axis (234) which extends orthogonal to a longitudinal axis (230) of the aiming guide, wherein in at least a cross-section orthogonal to the central axis (234) the tip (231) has a circularly curved periphery with a radius (R) and a centre located on the longitudinal axis (230).

2. The system according to claim 1, wherein the recess (3) extends through a proximal end of the screw head (2), the recess (3) also configured to receive a portion of a surgical tool to be releasably coupled thereto and having a substantially circular edge centered at an intersection of the through hole axis (7) and the screw axis (6).

3. The system according to claim 1, wherein the through hole (9) comprises an internal thread.

4. The system according to claim 2, wherein the recess (3) has a constriction (31) at the proximal end of the screw head (2) to facilitate a snap connection between the bone screw (1) and the surgical tool.

5. The system according to claim 4, wherein the recess (3) includes a depression (12)
traversing the constriction (31) and forming a wall portion forming a portion of one of a cylinder, cone and a prism an axis of which coincides with the through hole axis.

6. The system according to claim 1, wherein the channel axis intersects the screw axis at
the point at which the screw axis and the through hole axis intersect.

7. The system of any one of claims 1 to 6, in which the bone screw (1) is a first bone screw, and in which the system comprises a second bone screw (50) sized and shaped to be inserted through the through hole (9) of the first bone screw (1) along the through hole axis (7).

8. The system according to claim 1, further comprising a screwdriver for a bone screw,
the screw driver including a shaft extending longitudinally from a distal end to a proximal end, the distal end including a male connector configured to be coupled to the recess in the screw head of the first bone screw, the connector including a tip tapering towards the distal end of the screwdriver and two driving protrusions extending from opposite sides of the tip along a central axis extending orthogonally to the longitudinal axis of the screwdriver, wherein the driving protrusions engage the channel in the screw head of the first bone screw, and wherein the tip has a substantially circular periphery centered on the longitudinal axis.

9. The system according to claim 8, wherein the male connector further comprises an axial stop a distance T from the central axis towards the shaft so that the stop contacts the rear end of the first bone screw when the connector is coupled to the recess in the screw head.

10. The system according to claim 8, wherein the male connector further includes a nose projecting over the tip in a direction toward the distal end and at an acute angle with respect to the longitudinal axis of the screwdriver.

11. The system according to claim 7, further comprising a drill guide insertable into the through hole.

12. The system according to claim 11 , further comprising a screwdriver including a connector at a distal end thereof configured to receive the partially spherical screw head such that the first bone screw is pivotable between a first position in which the screw axis is substantially coaxial with a longitudinal axis of the screwdriver to a second position in which the through hole axis is coaxial with the longitudinal axis.

13. The system according to claim 12, wherein the screwdriver further includes a channel extending therethrough along the longitudinal axis thereof such that when the first bone screw is in the second position, the channel is configured to guide the second bone screw therethrough and into the through hole of the first bone screw.

## Patentansprüche

1. System, das eine Knochenschraube (1) und eine Zielführung (23) zum Bohren eines Lochs in einen Knochen umfasst;
wobei die Knochenschraube (1) umfasst:
einen Schraubenschaft (10), der sich längs entlang einer Schraubenachse (6) erstreckt;
einen Schraubenkopf (2), der sich von einem proximalen Ende des Schraubenschafts (10) erstreckt; und
ein Durchgangsloch (9), das eine Durchgangslochachse (7) definiert und sich durch den Schraubenkopf (2) erstreckt, wobei die Durchgangslochachse (7) die Schraubenachse (6) in einem spitzen Winkel α schneidet, wobei das Durchgangsloch (9) geeignet ist, eine zweite Schraube (50) aufzunehmen, und von einem ersten Ende an einem proximalen Ende des Schraubenkopfs (2) zu einem zweiten Ende, das sich zu einer äußeren Oberfläche der Knochenschraube (1) öffnet, konisch zuläuft;
wobei der Schraubenkopf (2) ein konkaves Auflager (14) hat, das einen Kanal (5) und eine zentral angeordnete Aussparung (3) umfasst, wobei der Kanal (5) an dem ersten Ende des Durchgangslochs (9) angeordnet ist und sich von der Außenoberfläche der Knochenschraube (2) quer durch den Schraubenkopf (2) entlang einer Kanalachse (101), die im Wesentlichen senkrecht zu der Schraubenachse (6) ist, erstreckt, wobei der Kanal (5) an dem proximalen Ende der Knochenschraube (1) offen ist;
wobei die Zielführung (23) zum Bohren eines Lochs mit einer Achse dient, die mit der Durchgangslochachse (7) des Durchgangslochs (9) in dem Schraubenkopf (2) der Knochenschraube (1) zusammenfällt;
und wobei die Zielführung (23) umfasst:
eine Führungshülse (26);
eine koaxiale Durchgangsbohrung (28); und
einen aufzunehmenden Verbinder (232), der abschließend an einem vorderen Ende (235) der Zielführung (23) angeordnet ist, um mit der Aussparung (3) in dem Schraubenkopf (2) zu koppeln, wobei der Verbinder (232) eine Spitze (231), deren Breite in Richtung des vorderen Endes (235) der Zielführung (23) abnimmt, und zwei Stifte (25) umfasst, die in beide Richtungen diametral über die Spitze (231) vorstehen und koaxial auf einer Mittelachse (234) angeordnet sind, die sich orthogonal zu einer Längsachse (230) der Zielführung erstreckt, wobei die Spitze (231) in wenigstens einem Querschnitt orthogonal zu der Mittelachse (234) einen kreisförmig gekrümmten Umfang mit einem Radius (R) und einer Mitte hat, welche sich auf der Längsachse (230) befindet.

2. System nach Anspruch 1, wobei die Aussparung (3) sich durch ein proximales Ende des Schraubenkopfs (2) erstreckt, die Aussparung (3) auch aufgebaut ist, um einen Abschnitt eines chirurgischen Werkzeugs aufzunehmen, das lösbar damit gekoppelt werden soll und eine im Wesentlichen kreisförmige Kante hat, die auf einen Schnittpunkt der Durchgangslochachse (7) und der Schraubenachse (6) zentriert ist.

3. System nach Anspruch 1, wobei das Durchgangsloch (9) ein Innengewinde umfasst.

4. System nach Anspruch 2, wobei die Aussparung (3) eine Verengung (31) an dem proximalen Ende des Schraubenkopfs (2) hat, um eine Schnappverbindung zwischen der Knochenschraube (1) und dem chirurgischen Werkzeug zu erleichtern.

5. System nach Anspruch 4, wobei die Aussparung (3) eine Vertiefung (12) umfasst, welche die Verengung (31) durchquert und einen Wandabschnitt bildet, der einen Abschnitt eines Zylinders, eines Kegels oder eines Prismas bildet, von dem eine Achse mit der Durchgangslochachse zusammenfällt.

6. System nach Anspruch 1, wobei die Kanalachse die Schraubenachse an dem Punkt schneidet, an dem die Schraubenachse und die Durchgangslochachse sich schneiden.

7. System nach einem der Ansprüche 1 bis 6, wobei die Knochenschraube (1) eine erste Knochenschraube ist, und wobei das System eine zweite Knochenschraube (50) umfasst, die derart bemessen und geformt ist, dass sie durch das Durchgangsloch (9) der ersten Knochenschraube (1) entlang der Durchgangslochachse (7) eingesetzt werden soll.

8. System nach Anspruch 1, das ferner einen Schraubenzieher für eine Knochenschraube umfasst, wobei der Schraubenzieher einen Schaft umfasst, der sich längs von einem distalen Ende zu einem proximalen Ende erstreckt, wobei das distale Ende einen aufzunehmenden Verbinder umfasst, der aufgebaut ist, um mit der Aussparung in dem Schraubenkopf der ersten Knochenschraube gekoppelt zu werden, wobei der Verbinder eine Spitze, die in Richtung des distalen Endes des Schraubenziehers konisch zuläuft, und zwei Antriebsvorsprünge umfasst, die sich von gegenüberliegenden Seiten der Spitze entlang einer Mittelachse erstrecken, welche sich orthogonal zu der Längsachse des Schraubenziehers erstreckt, wobei die Antriebsvorsprünge in den Kanal in dem Schraubenkopf der ersten Knochenschraube eingreifen, und wobei die Spitze einen im Wesentlichen kreisförmigen Umfang hat, der auf die Längsachse zentriert ist.

9. System nach Anspruch 8, wobei der aufzunehmende Verbinder ferner einen axialen Anschlag in einer Entfernung T von der Mittelachse in Richtung des Schafts umfasst, so dass der Anschlag das hintere Ende der ersten Knochenschraube berührt, wenn der Verbinder mit der Aussparung in dem Schraubenkopf gekoppelt wird.

10. System nach Anspruch 8, wobei der aufzunehmende Verbinder ferner eine Nase umfasst, die in einer Richtung in Richtung des distalen Endes und in einem spitzen Winkel in Bezug auf die Längsachse des Schraubenziehers über die Spitze hinaus vorsteht.

11. System nach Anspruch 7, das ferner eine Bohrführung umfasst, die in das Durchgangsloch einsetzbar ist.

12. System nach Anspruch 11, das ferner einen Schraubenzieher mit einem Verbinder an seinem distalen Ende umfasst, der aufgebaut ist, um den teilweise kugelförmigen Schraubenkopf derart aufzunehmen, dass die erste Knochenschraube zwischen einer ersten Position, in der die Schraubenachse im Wesentlichen koaxial mit einer Längsachse des Schraubenziehers ist, in eine zweite Position, in der die Durchgangslochachse koaxial mit der Längsachse ist, schwenkbar ist.

13. System nach Anspruch 12, wobei der Schraubenzieher ferner einen Kanal umfasst, der sich entlang seiner Längsachse derart durch ihn erstreckt, dass der Kanal aufgebaut ist, um die zweite Knochenschraube hindurch und in das Durchgangsloch der ersten Knochenschraube zu führen, wenn die erste Knochenschraube in der zweiten Position ist

## Revendications

1. Système comprenant une vis d'os (1) et un guide de visée (23) pour percer un trou dans un os ;
dans lequel la vis d'os (1) comprend :
une tige de vis (10) s'étendant longitudinalement le long d'un axe de vis (6) ;
une tête de vis (2) s'étendant depuis une extrémité proximale de la tige de vis (10) ; et
un trou traversant (9) définissant un axe de trou traversant (7) et s'étendant à travers la tête de vis (2), l'axe de trou traversant (7) croisant l'axe de vis (6) selon un angle aigu α, le trou traversant (9) étant adapté pour y recevoir une seconde vis (50) et se resserrant depuis une première extrémité au niveau d'une extrémité proximale de la tête de vis (2) vers une seconde extrémité s'ouvrant sur une surface extérieure de la vis d'os (1) ;
dans lequel la tête de vis (2) comporte un siège concave (14) comprenant un canal (5) et un évidement (3) situé au centre, le canal (5) étant disposé au niveau de la première extrémité du trou traversant (9) et s'étendant depuis la surface extérieure de la vis d'os (1) de manière transversale à travers la tête de vis (2) le long d'un axe de canal (101) qui est sensiblement perpendiculaire à l'axe de vis (6), le canal (5) étant ouvert au niveau de l'extrémité proximale de la vis d'os (1) ;
dans lequel le guide de visée (23) sert à percer un trou ayant un axe qui coïncide avec l'axe de trou traversant (7) du trou traversant (9) dans la tête de vis (2) de la vis d'os (1) ;
et dans lequel le guide de visée (23) comprend :
un manchon de guidage (26) ;
un trou traversant coaxial (28) ; et
un connecteur mâle (232) agencé de manière terminale au niveau d'une extrémité avant (235) du guide de visée (23) pour se coupler à l'évidement (3) dans la tête de vis (2), le connecteur (232) incluant une pointe (231) dont une largeur diminue vers l'extrémité avant (235) du guide de visée (23) et deux picots (25) se projetant diamétralement sur la pointe (231) dans chaque direction et agencés de manière coaxiale sur un axe central (234) qui s'étend de manière orthogonale à un axe longitudinal (230) du guide de visée, **caractérisé en ce que**, dans au moins une section transversale orthogonale à l'axe central (234), la pointe (231) présente une périphérie circulairement incurvée ayant un rayon (R) et un centre situé sur l'axe longitudinal (230).

2. Système selon la revendication 1, dans lequel l'évidement (3) s'étend à travers une extrémité proximale de la tête de vis (2), l'évidement (3) étant également configuré pour recevoir une partie d'un outil chirurgical à coupler de manière amovible à celui-ci et ayant un bord sensiblement circulaire centré au niveau d'une intersection de l'axe de trou traversant (7) et de l'axe de vis (6).

3. Système selon la revendication 1, dans lequel le trou traversant (9) comprend un filetage interne.

4. Système selon la revendication 2, dans lequel l'évidement (3) présente un étranglement (31) au niveau de l'extrémité proximale de la tête de vis (2) afin de faciliter une connexion par encliquetage entre la vis d'os (1) et l'outil chirurgical.

5. Système selon la revendication 4, dans lequel l'évidement (3) inclut un creux (12) traversant l'étranglement (31) et formant une partie de paroi formant une partie choisie parmi un cylindre, un cône et un prisme dont un axe coïncide avec l'axe de trou traversant.

6. Système selon la revendication 1, dans lequel l'axe de canal croise l'axe de vis au niveau du point où l'axe de vis et l'axe de trou traversant se croisent.

7. Système selon l'une des revendications 1 à 6, dans lequel la vis d'os (1) est une première vis d'os, et dans lequel le système comprend une seconde vis d'os (50) dimensionnée et façonnée pour être insérée dans le trou traversant (9) et la première vis d'os (1) le long de l'axe de trou traversant (7).

8. Système selon la revendication 1, comprenant en outre un tournevis pour une vis d'os, le tournevis incluant une tige s'étendant longitudinalement depuis une extrémité distale jusqu'à une extrémité proximale, l'extrémité distale incluant un connecteur mâle configuré pour être couplé à l'évidement dans la tête de vis de la première vis d'os, le connecteur incluant une pointe se resserrant vers l'extrémité distale du tournevis et deux saillies d'entraînement s'étendant depuis des côtés opposés de la pointe le long d'un axe central s'étendant de manière orthogonale à l'axe longitudinal du tournevis, dans lequel les saillies d'entraînement s'engagent avec le canal de la tête de vis de la première vis d'os, et dans lequel la pointe présente une périphérique sensiblement circulaire centrée sur l'axe longitudinal.

9. Système selon la revendication 8, dans lequel le connecteur mâle comprend en outre une butée axiale à une distance T de l'axe central vers la tige de telle sorte que la butée entre en contact avec l'extrémité arrière de la première vis d'os quand le connecteur est couplé à l'évidement dans la tête de vis.

10. Système selon la revendication 8, dans lequel le connecteur mâle inclut en outre un nez se projetant sur la pointe dans une direction vers l'extrémité distale et selon un angle aigu par rapport à l'axe longitudinal du tournevis.

11. Système selon la revendication 7, comprenant en outre un guide de foret pouvant être inséré dans le trou traversant.

12. Système selon la revendication 11, comprenant en outre un tournevis incluant un connecteur au niveau d'une extrémité distale de celui-ci configuré pour recevoir la tête de vis partiellement sphérique de telle sorte que la première vis d'os peut pivoter entre une première position dans laquelle l'axe de vis est sensiblement coaxial avec un axe longitudinal du tournevis jusqu'à une seconde position dans laquelle l'axe de trou traversant est coaxial avec l'axe longitudinal.

13. Système selon la revendication 12, dans lequel le tournevis inclut en outre un canal s'étendant à travers celui-ci le long de l'axe longitudinal de celui-ci de telle sorte que, lorsque la première vis d'os se trouve dans la seconde position, le canal est configuré pour guider la seconde vis d'os à travers celui-ci et dans le trou traversant de la première vis d'os.
